(19) Europäisches Patentamt
European Patent Office
Office européen des brevets

(11) EP 1 250 312 B1

(12) FASCICULE DE BREVET EUROPEEN

(45) Date de publication et mention de la délivrance du brevet:
**14.06.2006 Bulletin 2006/24**

(21) Numéro de dépôt: **00993711.1**

(22) Date de dépôt: **27.12.2000**

(51) Int Cl.:
*C07C 253/10* (2006.01) *C07C 253/12* (2006.01)
*B01J 31/24* (2006.01)

(86) Numéro de dépôt international:
**PCT/FR2000/003695**

(87) Numéro de publication internationale:
**WO 2001/049655 (12.07.2001 Gazette 2001/28)**

# (54) PROCEDE D'HYDROCYANATION DE COMPOSES ORGANIQUES INSATURES

VERFAHREN ZUR HYDROCYANIERUNG VON UNGESÄTTIGTEN ORGANISCHEN VERBINDUNGEN

METHOD FOR THE HYDROCYANATION OF UNSATURATED ORGANIC COMPOUNDS

(84) Etats contractants désignés:
**BE DE ES FR GB IT NL TR**

(30) Priorité: **30.12.1999 FR 9916714**

(43) Date de publication de la demande:
**23.10.2002 Bulletin 2002/43**

(73) Titulaire: **RHODIA POLYAMIDE INTERMEDIATES**
**69192 Saint-Fons (FR)**

(72) Inventeur: **BURATTIN, Paolo**
**F-69002 Lyon (FR)**

(74) Mandataire: **Esson, Jean-Pierre**
**Rhône-Poulenc Fibres**
**Service Brevets**
**Centre de Recherches des Carrières**
**B.P. 62**
**69192 Saint-Fons Cedex (FR)**

(56) Documents cités:
**US-A- 5 484 902** **US-A- 5 523 453**

- **MOINEAU J ET AL: "Palladium-catalyzed Heck reaction in perfluorinated solvents" TETRAHEDRON LETTERS,NL,ELSEVIER SCIENCE PUBLISHERS, AMSTERDAM, vol. 40, no. 43, 22 octobre 1999 (1999-10-22), pages 7683-7686, XP004179894 ISSN: 0040-4039 cité dans la demande**
- **MATHIVET T ET AL: "Unexpected Synthesis of a New Highly Fluorocarbon Soluble Phosphite for Biphasic Catalysis" TETRAHEDRON LETTERS, NL,ELSEVIER SCIENCE PUBLISHERS, AMSTERDAM, vol. 40, no. 20, 14 mai 1999 (1999-05-14), pages 3885-3888, XP004163771 ISSN: 0040-4039 cité dans la demande**
- **CHEMICAL ABSTRACTS, vol. 122, no. 1, 2 janvier 1995 (1995-01-02) Columbus, Ohio, US; abstract no. 9236b, CASALNUOVO, ALBERT L ET AL: "ligand electronic effects in asymmetric catalysis: Enhanced enantioselectivity in the asymmetric hydrocyanation of vinylarenes" page 1039; colonne 1; XP002144973 & JOURNAL OF THE AMERICAN CHEMICAL SOCIETY., vol. 116, no. 22, 1994, pages 9869-82, AMERICAN CHEMICAL SOCIETY, WASHINGTON, DC., US ISSN: 0002-7863**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 250 312 B1

Printed by Jouve, 75001 PARIS (FR)

## Description

La présente invention concerne un procédé d'hydrocyanation de composés organiques insaturés tels que des oléfines ou des composés organiques insaturés comprenant une fonction nitrile, pour la production de composés organiques mono ou polynitriles.

La réaction d'hydrocyanation d'une oléfine pour la fabrication de nitriles et plus particulièrement des dinitriles est connue et exploitée depuis de très longues années.

Cette réaction est notamment utilisée pour la production d'un grand intermédiaire chimique, l'adiponitrile, à partir du butadiène.

Ainsi, le brevet US 3 496 217 décrit un procédé de fabrication de l'adiponitrile par réaction du cyanure d'hydrogène sur du butadiène en présence d'un catalyseur à base de nickel lié à un ligand organique du type phosphine ou phosphite. La réaction est réalisée en milieu liquide monophasique.

Le brevet français n°2 338 253 décrit également un procédé de fabrication d'adiponitrile par hydrocyanation du butadiène. La réaction est réalisée en milieu liquide biphasique, le catalyseur étant contenu dans une phase aqueuse. Ce procédé permet de récupérer l'adiponitrile dans la phase organique exempt de catalyseur et donc de métal. Le catalyseur décrit est également un catalyseur à base d'un métal tel que le nickel associé à un ligand du type phosphine. Toutefois, ce ligand comprend des radicaux sulfonates permettant de rendre soluble ou dispersable le catalyseur dans l'eau.

De nombreux autres brevets ont été publiés sur des structures particulières de ligands du type phosphites ou phosphines tant dans le domaine de la réaction monophasique que biphasique. Le brevet US 5 523 453, par exemple, décrit un procédé d'hydrocyanation d'oléfines avec cyanure d'hydrogène en présence d'un catalyseur comprenant un élément métallique choisi par les métaux de transition (comme le nickel) et un ligand organophosphoré comprenant des atomes de fluor. La réaction peut être mise en oeuvre dans un solvent comme le benzène, xylène, acetonitrile ou benzonitrile ou en absence de solvant.

Ces ligands appartenant à la famille des phosphites ou phosphines génèrent soit des problèmes pour leur extraction et récupération du milieu réactionnel soit une capacité limitée d'échange de catalyseur entre les phases.

Un des buts de l'invention est de répondre à ces problèmes en proposant un procédé d'hydrocyanation en milieu liquide à base d'un catalyseur comprenant un nouveau type de ligand.

A cet effet, l'invention propose un procédé d'hydrocyanation d'un composé hydrocarboné comprenant au moins une insaturation éthylénique ou acétylénique par réaction en milieu liquide avec le cyanure d'hydrogène en présence d'un catalyseur.

Selon l'invention, le catalyseur comprend un métal de transition associé à un ligand organophosphoré comprenant des atomes de fluor. En outre, la réaction est mise en oeuvre en presence d'un solvant fluoré dans lequel le catalyseur est soluble. Ainsi, le catalyseur est au moins partiellement extractible du milieu réactionnel par ce solvant liquide dans lequel les composés hydrocarbonés et les composés hydrocyanés formés par la réaction ne sont pas solubles.

Selon une autre caractéristique préférée de l'invention, la teneur pondérale en fluor dans le ligand organophosphoré est au moins égale à 10 %, avantageusement comprise entre 20%et90%.

Selon une nouvelle caractéristique de l'invention, le catalyseur correspond à la formule générale (I):

$$M\,[L_f]_s$$

Dans laquelle:

M est un métal de transition choisi dans le groupe comprenant le nickel, le fer, le palladium.
$L_f$ représente le ligand organophosphoré choisi dans le groupe comprenant les phosphines, les phosphinites, les phosphonites, les phosphites ou leurs mélanges, ledit ligand comprenant au moins un radical fluoré,
s représente un nombre compris entre 1 et 6 (bornes incluses)

Selon un mode de réalisation préféré de l'invention, le radical fluoré est:

- un radical alkyl de formule (II):

$$-C_nH_{2n+1-m}F_m$$

dans laquelle :

n est un nombre entier compris entre 1 et 8
m est un nombre entier déterminé par l'inéquation:

$$\frac{m}{2n+1} > 0{,}25$$

- ou un radical polyéther de formule (III):

$$[-O-C_pH_{2p-q}F_q]_x-$$

dans laquelle:

p est un nombre entier compris entre 1 et 8,
x est supérieur ou égal à 1 de préférence < 20, avantageusement < 10, et q est un nombre entier déterminé par l'inéquation:

$$\frac{q}{2p} > 0,25$$

Selon une caractéristique préférentielle, le ligand comprend au moins un radical comprenant un groupe phényl substitué par au moins un radical fluoré correspondant aux formules générales (II) et (III).

Comme ligands convenables pour l'invention on peut citer les composés décrits dans les articles dont les références sont:

D. RUTHERFORD et al, Catal. Today 42 (1998) 381-388
T. MATHIVET et al, Tet. Lett. 40 (1999) 3885-3888
J. MOINEAU et al, Tet. Lett. 40 (1999) 7683-7686
B. RICHTER et al, J. Mol. Catal. A : Chem. 145 (1999) 317-321

Les catalyseurs convenables pour l'invention peuvent être préparés selon les méthodes habituelles de préparation des catalyseurs comprenant un élément métallique associé à un ligand, par exemple par addition d'un composé dudit métal dans une solution du ligand. Le catalyseur peut également être formé in situ dans le milieu réactionnel d'hydrocyanation par addition dans ledit milieu contenant le ligand, d'un composé du métal de transition.

Des exemples de procédés de fabrication de tels catalyseurs avec des ligands analogues mais non fluorés sont décrits dans les brevets US 3496217 et FR 2338253.

Selon la solubilité du ligand dans le composé organique à hydrocyaner ou un solvant dudit composé, la réaction peut être réalisée soit en milieu monophasique soit en milieu polyphasique. En outre, il est également possible d'utiliser un solvant du catalyseur, immiscible à basse température avec le composé organique à hydrocyaner, mais devenant miscible quand la température augmente. Ce mode de réalisation permet d'effectuer la réaction en milieu monophasique et par refroidissement du milieu d'extraire le catalyseur dans son solvant quand le milieu redevient biphasique.

Dans un mode de réalisation préféré, le solvant d'extraction du catalyseur ou solvant du catalyseur est de préférence un solvant constitué par un composé comprenant des atomes de fluor, appelé généralement solvant fluoré. Toutefois, d'autres solvants peuvent être utilisés sans sortir du cadre de l'invention.

En effet, tout solvant qui solubilise le catalyseur et dans lequel, dans certaines conditions, les composés à hydrocyaner et les composés résultant de la réaction d'hydrocyanation sont insolubles ou solubles à une concentration en poids inférieure à 30% est convenable pour l'invention.

A titre d'exemple de solvants fluorés on peut citer les perfluoroalcanes, perfluoroalkylethers, perfluoroalkylamines, perfluoropolyalkyléthers.

On peut définir comme solvants fluorés convenables pour l'invention les composés organiques dans lesquels le taux d'atomes de fluor représente au moins 20 % en poids du poids total du solvant.

Selon l'invention, le composé à hydrocyaner peut être mis en oeuvre tel quel et donc forme la phase liquide dans laquelle est dissous le nitrile formé. Il est également possible de dissoudre ce composé dans un solvant organique tiers. Comme solvants convenables notamment pour la mise en oeuvre de système polyphasique, on peut citer les hydrocarbures tels que le toluène, l'hexane, le benzène, les xylènes, l'heptane, le cyclohexane, le pentane.

L'invention s'applique notamment à l'hydrocyanation des oléfines, des nitriles insaturés. Ainsi, une application préférée de l'invention est la synthèse de l'adiponitrile qui consiste à transformer le butadiène en 3-pentènenitrile, puis dans une seconde étape à hydrocyaner le 3-pentènenitrile en adiponitrile.

L'invention concerne plus particulièrement un procédé d'hydrocyanation d'une oléfine comprenant au moins deux insaturations éthyléniques conjuguées ou non, par exemple le butadiène, par réaction avec le cyanure d'hydrogène.

Cette réaction est réalisée en milieu liquide monophasique ou polyphasique, avantageusement biphasique, c'est à dire une phase organique contenant l'oléfine, et une seconde phase non miscible dans laquelle le catalyseur d'hydrocyanation est solubilisé.

Cette réaction est généralement réalisée en plusieurs étapes. Ainsi, dans une première étape, une seule insaturation éthylénique de l'oléfine est hydrocyanée pour produire un nitrile insaturé. Généralement, plusieurs isomères linéaires et ramifiés de nitriles insaturés sont produits.

Dans un objectif de favoriser la formation d'un isomère particulier, par exemple l'isomère linéaire, une deuxième étape appelée "isomérisation" est mise en oeuvre. Le milieu réactionnel peut être identique mais les conditions opératoires et la concentration en cyanure d'hydrogène sont adaptées.

Enfin, dans une troisième étape, la seconde liaison éthylénique est hydrocyanée pour produire un dinitrile. Dans le cas du butadiène, l'adiponitrile est ainsi obtenu en fin de troisième étape.

Le procédé de l'invention s'applique aux trois étapes décrites ci-dessus, et plus particulièrement aux première et deuxième étapes comme cela est illustré ci-après.

Les première et deuxième étapes sont réalisées avantageusement à une température comprise entre 30°C et 150°C, avec une quantité de catalyseur exprimée en nickel comprise entre $10^{-4}$ et 1 mole de métal par litre de milieu réactionnel, avantageusement entre 0,005 et 0,5 mole/l.

Egalement, la quantité de ligand fluoré ajouté

dans le milieu réactionnel est choisie de telle sorte que le nombre de moles de ce composé rapporté à une mole de métal élémentaire soit compris entre 0,5 et 2000, de préférence de 2 à 300.

On peut également utiliser des cocatalyseurs tels que des acides de Lewis. Par acide de Lewis, il faut comprendre les composés accepteurs de doublets électroniques.

Ainsi, à titre d'exemple, on peut citer les acides de Lewis choisis parmi les composés des éléments des groupes Ib, IIb, IIIa, IIIb, IVa, IVb, Va, Vb, VIb, VIIb et VIII de la Classification périodique des éléments, dans la mesure où lesdits composés sont au moins partiellement solubles et stables dans le milieu utilisé. Ces composés sont le plus souvent des sels, notamment des halogénures, de préférence chlorures et bromures, des sulfates, des carboxylates et des phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, les chlorures ou bromures des éléments des terres rares comme le lanthane, le cérium, le praséodyme, le néodyme, le samarium, l'europium, le gadolinium, le terbium, le dysprosium, l'holmium, l'erbium, le thulium, l'ytterbium et le lutétium, le chlorure de cobalt, le chlorure ferreux, le chlorure d'yttrium.

On peut bien entendu mettre en oeuvre des mélanges de plusieurs acides de Lewis.

Le cocatalyseur acide de Lewis mis en oeuvre représente généralement de 0,01 à 5 moles par mole de métal élémentaire du catalyseur, de préférence de 1 à 10 moles par mole.

La solution catalytique utilisée pour l'hydrocyanation selon l'invention peut être préparée avant son introduction dans la zone de réaction, par exemple par addition à la solution dans le solvant utilisé du ligand organophosphoré de formule (I), de la quantité appropriée de composé du métal de transition choisi, de l'acide de Lewis et éventuellement du réducteur. Il est également possible de préparer la solution catalytique "in situ" par simple mélange de ces divers constituants.

La réaction peut être réalisée sans tiers solvant, c'est à dire sans solvant de l'oléfine ou du composé à hydrocyaner. Il peut être toutefois avantageux d'utiliser un solvant organique inerte, non miscible au solvant fluoré, qui pourra permettre l'extraction ultérieure des nitriles formés.

Selon le type de ligand utiliser ou de solvant, la réaction d'hydrocyanation est réalisée soit en milieu monophasique soit en milieu biphasique. Les procédés de récupération ou séparation du catalyseur seront différents selon le cas. Ainsi, dans le cas d'un milieu monophasique, le catalyseur est extrait par un tiers solvant. Dans le cas d'un milieu biphasique, le catalyseur se trouvant majoritairement dans la phase ne contenant pas le nitrile formé, il est donc récupéré par séparation des phases.

Le procédé de l'invention peut être mis en oeuvre de manière continue ou discontinue.

Le cyanure d'hydrogène mis en oeuvre peut être préparé à partir des cyanures métalliques, notamment le cyanure de sodium ou des cyanhydrines.

Le cyanure d'hydrogène est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le ligand fluoré, le composé de métal de transition, les éventuels réducteur et solvant, l'acide de Lewis, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Lorsque la réaction (dont on peut suivre l'évolution par prélèvements et dosage) est terminée, le mélange réactionnel est soutiré après refroidissement et les produits de la réaction sont séparés du catalyseur par les procédés décrit précédemment.

La solution contenant le catalyseur ou celui-ci peut alors être recyclé dans une nouvelle réaction d'hydrocyanation.

Dans le cadre d'une mise en oeuvre du procédé en continu d'un système biphasique, seule la phase organique peut être soutirée, tandis que la phase catalytique demeure dans le réacteur.

L'invention est illustrée par les exemples donnés ci-dessous uniquement à titre indicatif et sans limitation de la portée de l'invention.

**<u>EXEMPLE 1 :</u> Préparation d'un catalyseur conforme à l'invention**

Dans un ballon de 500 mL muni d'un barreau aimanté et purgé à l'argon, on charge 12,7 g de ligand fluoré $L_f$ de formule:

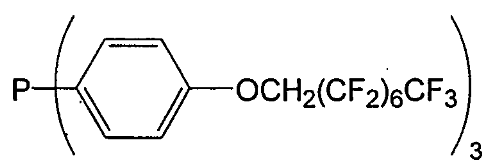

188,6 g de perfluoro(méthylcyclohexane) et 87,0 g de toluène sont également ajoutés. On obtient ainsi un système biphasique.

Sous agitation et à température ambiante, 800 mg de Ni

$(COD)_2$ (COD = 1,5-cyclooctadiène) sont additionnés. Le catalyseur constitué du Ni à l'état d'oxydation 0 et du ligand organophosphoré est ainsi formé. Un dosage du nickel et du phosphore sur un prélèvement de la phase inférieure fluorée, de coloration rouge intense, montre que celle-ci contient 80% en poids dudit catalyseur.

**EXEMPLE 2 : Isomérisation du 2-méthyl-3-butènenitrile (2M3BN) en 3-pentène nitrile (système biphasique)**

Sous atmosphère d'argon, 1,910 g de la phase fluorée de l'Exemple 1 sont introduits dans un réacteur en verre de 4 ml muni d'un barreau aimanté. Après ajout de 0,910 g de toluène et de 0,774 g d'un mélange de pentènenitriles contenant environ 80% mol. de 2-méthyl-3-butènenitrile (2M3BN), le tube est fermé et porté sous agitation à 100°C pendant 6 heures, puis à 120°C pendant 3h, puis encore à 100°C pendant 6h. Après retour à température ambiante et décantation, un prélèvement de la phase organique est analysé par chromatographie en phase gazeuse.
Les résultats obtenus sont:

conversion du 2M3BN égale à 94 %
sélectivité en 3 et 4-pentènenitrile (3PN et 4PN) égale à 89%.

Un dosage du nickel et du phosphore sur un prélèvement de la phase fluorée montre que celle-ci contient environ 85% en poids du catalyseur engagé.

**EXEMPLE 3 : Isomérisation du 2-méthyl-3-butènenitrile (2M3BN) en 3-pentène nitrile (système monophasique)**

Sous atmosphère d'argon, 1,79 g de la phase fluorée de l'Exemple 1 sont introduits dans un tube en saphir (tube transparent pressurisable). Après ajout de 0,850 g de toluène et de 0,790 g d'un mélange de pentènenitriles contenant environ 80% mol. de 2M3BN, le tube est préssurisé à 20 bar d'azote, fermé puis chauffé à 150°C. Dans ces conditions de température et de pression, on obtient un système monophasique parfaitement homogène et limpide. Après 3h à 150°C, retour à température ambiante et décantation, un prélèvement de la phase organique supérieure est analysé par chromatographie en phase gazeuse.
Les résultats obtenus sont:

conversion du 2M3BN égale à 16 %
sélectivité en 3 et 4-pentènenitrile (3PN et 4PN) égale à 84,5%.

Dans les exemples ci-dessus, le taux de conversion correspond au nombre de moles de 2M3BN transformées exprimé en pourcentage, la sélectivité est le pourcentage en mole de 2M3BN transformé en 3PN et 4PN calculé par rapport au nombre de moles de 2M3BN transformés.

**EXEMPLE 4 : Hydrocyanation du 3-pentène nitrile en adiponitrile (système biphasique)**

Sous atmosphère d'argon, 1,80 g de méthylcyclohexane perfluoré dégazé sont introduits dans un tube Shott (tube transparent pressurisable). On ajoute de 0,9 g de toluène, 76 mg de 3-pentène nitrile, 6 mg de $Ni(cod)_2$ 140 mg de ligand $L_F$ décrit à l'exemple 1, de la cyanhydrine de l'acétone à 30 équivalents par rapport au nickel et du triphényl de bore( 1 équivalent par rapport au nickel). Le mélange est chauffé à 65°C, sous agitation pendant 3 heures. Après 3h à 65°C, et refroidissement du mélange à température ambiante, un prélèvement de la phase organique supérieure est analysé par chromatographie en phase gazeuse.
Les résultats obtenus sont:

conversion du 3-pentène nitrile égale à 15 %
sélectivité en dinitriles égale à 16 %.

Rapport dinitriles linéaires / dinitriles totaux : 40 % en mole

**EXEMPLE 5 : Hydrocyanation du 3-pentène nitrile en adiponitrile (système monophasique)**

Sous atmosphère d'argon, 2 g de toluène perfluoré dégazé sont introduits dans un tube Shott (tube transparent pressurisable). On ajoute 74 mg de 3-pentène nitrile (30 équivalents par rapport au nickel), 6 mg de $Ni(cod)_2$ 140 mg de ligand $L_F$ décrit à l'exemple 1 ( 4,5 équivalents par rapport au nickel), de la cyanhydrine de l'acétone à 30 équivalents par rapport au nickel et du chlorure de zinc ( un équivalent par rapport au nickel). Le mélange est chauffé à 65°C, sous agitation pendant 3 heures. Dans ces conditions le mélange est homogène et comprend une seule phase. Après 3h à 65°C, et refroidissement du mélange à température ambiante, un prélèvement de celui-ci est analysé par chromatographie en phase gazeuse.
Les résultats obtenus sont:

conversion du 3-pentène nitrile égale à 7 %
sélectivité en dinitriles égale à 8 %.

Rapport dinitriles linéaires / dinitriles totaux : 59 % en mole

## Revendications

1. Procédé d'hydrocyanation d'un composé hydrocarboné comprenant au moins une insaturation éthylénique ou acétylénique par réaction en milieu liquide avec le cyanure d'hydrogène en présence d'un catalyseur, **caractérisé en ce que** le catalyseur comprend un élément métallique choisi parmi les métaux de transition et un ligand organophosphoré comprenant des atomes de fluor et **en ce que** la réaction est mise en oeuvre en présence d'un solvant fluoré dans lequel le catalyseur est soluble.

2. Procédé selon la revendication 1, **caractérisé en ce que** la concentration pondérale en élément fluor dans la formule du solvant fluoré représente au moins 20% du poids total dudit composé.

3. Procédé selon la revendication 2, **caractérisé en ce que** le solvant fluoré est choisi dans le groupe comprenant les perfluoroalcanes, perfluoroalkylethers, perfluoroalkylamines, perfluoropolyalkyléthers

4. Procédé selon l'une des revendications 1 à 3, **caractérisé en ce que** la teneur pondérale en fluor dans le ligand organophosphoré est au moins égale à 10 %.

5. Procédé selon la revendication 4, **caractérisé en ce que** la teneur pondérale en fluor dans le ligand organophosphoré est comprise entre 20 % et 90 %.

6. Procédé selon l'une des revendications précédentes, **caractérisé en ce que** l'élément métallique est choisi dans le groupe comprenant le nickel, le fer, le palladium.

7. Procédé selon l'une des revendications précédentes, **caractérisé en ce que** le catalyseur correspond à la formule générale (I):

$$M\,[L_f]_s$$

Dans laquelle:

M est un métal de transition choisi dans le groupe comprenant le nickel, le fer, le palladium.
$L_f$ représente le ligand organophosphoré choisi dans le groupe comprenant les phosphines, les phosphinites, les phosphonites, les phosphites ou leurs mélanges, ledit ligand comprenant au moins un radical fluoré,
s représente un nombre compris entre 1 et 6.

8. Procédé selon la revendication 7, **caractérisé en ce que** le radical fluorés est:

- un radical alkyl de formule (II):

$$-C_nH_{2n+1-m}F_m$$

dans laquelle :

n est un nombre entier compris entre 1 et 8
m est un nombre entier déterminé par l'inéquation

$$\frac{m}{2n+1} > 0,25$$

- ou un radical polyéther de formule (III):

$$[-O-C_pH_{2p-q}F_q]_x-$$

dans laquelle:

p est un nombre entier compris entre 1 et 8,
x est supérieur ou égal à 1, et
q est un nombre entier déterminé par l'inéquation

$$\frac{q}{2p} > 0,25$$

9. Procédé selon la revendication 7 ou 8, **caractérisé en ce que** le ligand comprend au moins un radical comprenant un groupe phényl substitué par au moins un radical fluoré correspondant aux formules générales (II) et (III).

10. Procédé selon l'une des revendications précédentes, **caractérisé en ce que** la réaction est effectuée en milieu monophasique.

11. Procédé selon la revendication 10, **caractérisé en ce que** le solvant fluoré, solvant du catalyseur est miscible à la phase comprenant le composé à hydrocyaner à la température d'hydrocyanation et immiscible avec ladite phase à température inférieure.

12. Procédé selon l'une quelconque des revendications 1 à 9, **caractérisé en ce que** la réaction est réalisée en milieu polyphasique.

13. Procédé selon l'une des revendications 1 à 12, **caractérisé en ce que** le solvant d'extraction du catalyseur est un solvant fluoré.

14. Procédé selon l'une des revendications précédentes, **caractérisé en ce qu'**un acide de LEWIS est ajouté dans le milieu réactionnel.

15. Procédé selon l'une des revendications précédentes, **caractérisé en ce qu'**un solvant du composé à hydrocyaner est utilisé

**16.** Procédé selon l'une des revendications précédentes, **caractérisé en ce que** le composé à hydrocyaner est une oléfine.

**17.** Procédé selon l'une des revendications précédentes, **caractérisé en ce que** la réaction d'hydrocyanation est une hydrocyanation d'une insaturation éthylénique ou acétylénique du composé à hydrocyaner.

**18.** Procédé selon l'une des revendications précédentes, **caractérisé en ce que** la réaction d'hydrocyanation est une isomérisation de composés mononitriles insaturés ramifiés en composés mononitriles insaturés linéaires.

**19.** Procédé selon l'une des revendications précédentes, **caractérisé en ce que** le composé à hydrocyaner est le butadiène.

**20.** Procédé selon la revendication 18, **caractérisé en ce que** le composé mononitrile insaturé est le 2-méthyl-3-butènenitrile.

**21.** Procédé selon l'une des revendications précédentes, **caractérisé en ce que** la quantité molaire de ligand par rapport à une mole d'élément métallique est comprise entre 0,5 et 2000.

**22.** Procédé selon l'une des revendications précédentes, **caractérisé en ce que** la concentration en élément métallique par litre de milieu réactionnel est comprise entre $10^{-4}$ et 1 mole de métal par litre de milieu réactionnel.

## Claims

**1.** Process for the hydrocyanation of a hydrocarbonaceous compound comprising at least one ethylenic or acetylenic unsaturation by reaction in a liquid medium with hydrogen cyanide in the presence of a catalyst, **characterized in that** the catalyst comprises a metal element chosen from transition metals and an organophosphorus ligand comprising fluorine atoms and **in that** the reaction is carried out in the presence of a fluorinated solvent in which the catalyst is soluble.

**2.** Process according to Claim 1, **characterized in that** the concentration by weight of fluorine element in the formula of the fluorinated solvent represents at least 20% of the total weight of the said compound.

**3.** Process according to Claim 2, **characterized in that** the fluorinated solvent is chosen from the group consisting of perfluoroalkanes, perfluoroalkyl ethers, perfluoroalkylamines and perfluoropolyalkyl ethers.

**4.** Process according to one of Claims 1 to 3, **characterized in that** the content by weight of fluorine in the organophosphorus ligand is at least equal to 10%.

**5.** Process according to Claims 4, **characterized in that** the content by weight of fluorine in the organophosphorus ligand is between 20% and 90%.

**6.** Process according to one of the preceding claims, **characterized in that** the metal element is chosen from the group consisting of nickel, iron and palladium.

**7.** Process according to one of the preceding claims, **characterized in that** the catalyst corresponds to the general formula (I):

$$M[L_f]_s$$

in which:

M is a transition metal chosen from the group consisting of nickel, iron and palladium,
$L_f$ represents the organophosphorus ligand chosen from the group consisting of phosphines, phosphinites, phosphonites, phosphites and their mixtures, the said ligand comprising at least one fluorinated radical,
s represents a number between 1 and 6.

**8.** Process according to Claim 7, **characterized in that** the fluorinated radical is:

- an alkyl radical of formula (II):

$$-C_nH_{2n+1-m}F_m$$

in which:

n is an integer between 1 and 8
m is an integer determined by the inequation:

$$\frac{m}{2n+1} > 0.25$$

- or a polyether radical of formula (III):

$$[-O-C_pH_{2p-q}F_q]_x-$$

in which:

p is an integer between 1 and 8,
x is greater than or equal to 1, and
q is an integer determined by the inequation:

$$\frac{q}{2p} > 0.25$$

**9.** Process according to Claim 7 or 8, **characterized in that** the ligand comprises at least one radical comprising a phenyl group substituted by at least one fluorinated radical corresponding to the general formulae (II) and (III).

**10.** Process according to one of the preceding claims, **characterized in that** the reaction is carried out in a single-phase medium.

**11.** Process according to Claim 10, **characterized in that** the fluorinated solvent, solvent of the catalyst, is miscible with the phase comprising the compound to be hydrocyanated at the hydrocyanation temperature and immiscible with the said phase at a lower temperature.

**12.** Process according to any one of Claims 1 to 9, **characterized in that** the reaction is carried out in a multiphase medium.

**13.** Process according to one of Claims 1 to 12, **characterized in that** the solvent for extraction of the catalyst is a fluorinated solvent.

**14.** Process according to one of the preceding claims, **characterized in that** a Lewis acid is added to the reaction medium.

**15.** Process according to one of the preceding claims, **characterized in that** a solvent of the compound to be hydrocyanated is used.

**16.** Process according to one of the preceding claims, **characterized in that** the compound to be hydrocyanated is an olefin.

**17.** Process according to one of the preceding claims, **characterized in that** the hydro-cyanation reaction is a hydrocyanation of an ethylenic or acetylenic unsaturation of the compound to be hydrocyanated.

**18.** Process according to one of the preceding claims, **characterized in that** the hydro-cyanation reaction is an isomerization of branched unsaturated mononitrile compounds to linear unsaturated mononitrile compounds.

**19.** Process according to one of the preceding claims, **characterized in that** the compound to be hydrocyanated is butadiene.

**20.** Process according to Claim 18, **characterized in that** the unsaturated mononitrile compound is 2-methyl-3-butenenitrile.

**21.** Process according to one of the preceding claims, **characterized in that** the molar amount of ligand with respect to one mol of metal element is between 0.5 and 2 000.

**22.** Process according to one of the preceding claims, **characterized in that** the concentration of metal element per litre of reaction medium is between $10^{-4}$ and 1 mol of metal per litre of reaction medium.

**Patentansprüche**

**1.** Verfahren zur Hydrocyanierung einer Kohlenwasserstoff-Verbindung, die mindestens eine ethylenische oder acetylenische Unsättigung umfaßt, durch Reaktion im flüssigen Medium mit Cyanwasserstoff in Anwesenheit eines Katalysators, **dadurch gekennzeichnet, daß** der Katalysator ein metallisches Element umfaßt, ausgewählt unter den Übergangsmetallen und einem Organophosphor-Liganden, der Fluoratome umfaßt, und dadurch, daß die Reaktion in Anwesenheit eines fluorierten Lösungsmittels durchgeführt wird, in dem der Katalysator löslich ist.

**2.** Verfahren nach Anspruch 1, **dadurch gekennzeichnet, daß** die gewichtsmäßige Konzentration an Element Fluor in der Formel des fluorierten Lösungsmittels mindestens insgesamt 20 Gew.-% der genannten Verbindung ausmacht.

**3.** Verfahren nach Anspruch 2, **dadurch gekennzeichnet, daß** das fluorierte Lösungsmittel aus der Gruppe gewählt wird, die Perfluoralkane, Perfluoralkylether, Perfluoralkylamine, und Perfluorpolyalkylether umfaßt.

**4.** Verfahren nach einem der Ansprüche 1 bis 3, **dadurch gekennzeichnet, daß** der gewichtsmäßige Gehalt an Fluor in dem Organophosphor-Liganden mindestens gleich 10 % beträgt.

**5.** Verfahren nach Anspruch 4, **dadurch gekennzeichnet, daß** der gewichtsmäßige Gehalt an Fluor in dem Organophosphor-Liganden zwischen einschließlich 20 % und 90 % beträgt.

**6.** Verfahren nach einem der vorstehenden Ansprüche, **dadurch gekennzeichnet, daß** das metallische Element aus der Gruppe gewählt wird, die Nickel, Eisen und Palladium umfaßt.

**7.** Verfahren nach einem der vorstehenden Ansprüche, **dadurch gekennzeichnet, daß** der Katalysator der

allgemeinen Formel (I) entspricht: $M[L_f]_s$ (I)
in der
M ein Übergangsmetall darstellt, gewählt aus der Gruppe, die Nikkel, Eisen und Palladium umfaßt,
$L_f$ einen Organophosphor-Liganden darstellt, gewählt aus der Gruppe, welche die Phosphine, die Phosphinite, die Phosphonite, die Phosphite oder ihre Mischungen umfaßt, wobei der genannte Ligand mindestens einen fluorierten Rest umfaßt,
s eine Zahl zwischen einschließlich 1 und 6 darstellt.

**8.** Verfahren nach Anspruch 7, **dadurch gekennzeichnet, daß** der fluorierte Rest ist:

- ein Rest Alkyl der Formel (II)

$$-C_nH_{2n+1-m}F_m$$

in der
n eine ganze Zahl zwischen einschließlich 1 und 8 ist;
m eine ganze Zahl ist, bestimmt durch die Ungleichung

$$\frac{m}{2n+1} > 0,25$$

- oder ein Rest Polyether der Formel (III)

$$[-O-C_pH_{2p-q}F_q]_x-$$

in der
p eine ganze Zahl zwischen einschließlich 1 und 8 ist;
x größer oder gleich 1 ist; und
q eine ganze Zahl ist, bestimmt durch die Ungleichung

$$\frac{q}{2p} > 0,25$$

**9.** Verfahren nach Anspruch 7 oder 8, **dadurch gekennzeichnet, daß** der Ligand mindestens einen Rest umfaßt, umfassend eine Gruppe Phenyl, die substituiert ist durch mindestens einen fluorierten Rest, der den allgemeinen Formeln (II) und (III) entspricht.

**10.** Verfahren nach einem der vorstehenden Ansprüche, **dadurch gekennzeichnet, daß** die Reaktion im monophasischen Medium durchgeführt wird.

**11.** Verfahren nach Anspruch 10, **dadurch gekennzeichnet, daß** das fluorierte Lösungsmittel, das Lösungsmittel des Katalysators, mit der Phase mischbar ist, welche die bei der Temperatur der Hydrocyanierung zu hydrocyanierende Verbindung umfaßt und unmischbar ist mit der genannten Phase bei niedriger Temperatur.

**12.** Verfahren nach einem der Ansprüche 1 bis 9, **dadurch gekennzeichnet, daß** die Reaktion im polyphasischen Medium durchgeführt wird.

**13.** Verfahren nach einem der Ansprüche 1 bis 12, **dadurch gekennzeichnet, daß** das Lösungsmittel zur Extraktion des Katalysators ein fluoriertes Lösungsmittel ist.

**14.** Verfahren nach einem der vorstehenden Ansprüche, **dadurch gekennzeichnet, daß** eine LEWIS-Säure zu dem Reaktionsmedium gegeben wird.

**15.** Verfahren nach einem der vorstehenden Ansprüche, **dadurch gekennzeichnet, daß** ein Lösungsmittel der zu hydrocyanierenden Verbindung verwendet wird.

**16.** Verfahren nach einem der vorstehenden Ansprüche, **dadurch gekennzeichnet, daß** die zu hydrocyanierende Verbindung ein Olefin ist.

**17.** Verfahren nach einem der vorstehenden Ansprüche, **dadurch gekennzeichnet, daß** die Reaktion der Hydrocyanierung eine Hydrocyanierung einer ethylenischen oder acetylenischen Unsättigung der zu hydrocyanierenden Verbindung ist.

**18.** Verfahren nach einem der vorstehenden Ansprüche, **dadurch gekennzeichnet, daß** die Reaktion der Hydrocyanierung eine Isomerisierung von verzweigten, ungesättigten Mononitril-Verbindungen zu linearen, ungesättigten Mononitril-Verbindungen ist.

**19.** Verfahren nach einem der vorstehenden Ansprüche, **dadurch gekennzeichnet, daß** die zu hydrocyanierende Verbindung Butadien ist.

**20.** Verfahren nach Anspruch 18, **dadurch gekennzeichnet, daß** die ungesättigte Mononitril-Verbindung 2-Methyl-3-butennitril ist.

**21.** Verfahren nach einem der vorstehenden Ansprüche, **dadurch gekennzeichnet, daß** die molare Menge von Ligand, im Verhältnis zu einem Mol metallischem Element, zwischen einschließlich 0,5 und 2000 beträgt.

**22.** Verfahren nach einem der vorstehenden Ansprüche, **dadurch gekennzeichnet, daß** die Konzentration an metallischem Element pro Liter Reaktionsmedium zwischen einschließlich $10^{-4}$ und 1 mol Metall pro Liter Reaktionsmedium beträgt.